Europäisches Patentamt

European Patent Office

Office européen des brevets

(19)

(11) **EP 1 005 906 B1**

(12) **FASCICULE DE BREVET EUROPEEN**

(45) Date de publication et mention
de la délivrance du brevet:
**15.09.2004 Bulletin 2004/38**

(51) Int Cl.[7]: **B01J 21/10**, B01J 23/02,
B01J 37/03, C07C 319/08

(21) Numéro de dépôt: **99402816.5**

(22) Date de dépôt: **12.11.1999**

(54) **Préparation des thiols en présence des catalyseurs comprenant de l'oxyde de zirconium et de l'oxyde de magnésium**

Herstellung von Thiolen mit Zirkonoxid und Magnesiumoxid enthaltenden Katalysatoren

Preparation of thiols using catalysts containing zirconium oxide and magnesium oxide

(84) Etats contractants désignés:
**BE FR NL**

(30) Priorité: **01.12.1998 FR 9815108**

(43) Date de publication de la demande:
**07.06.2000 Bulletin 2000/23**

(73) Titulaire: **Atofina**
**92800 Puteaux (FR)**

(72) Inventeurs:
 • **Boudjemaa, Fabien**
   **69100 Villeurbanne (FR)**
 • **Lacroix, Michel**
   **69009 Lyon (FR)**
 • **Forquy, Christian**
   **64360 Monein (FR)**

(74) Mandataire: **Granet, Pierre**
**Atofina,**
**Département Propriété Industrielle,**
**4-8, cours Michelet,**
**La Défense 10**
**92091 Paris La Défense Cedex (FR)**

(56) Documents cités:
**EP-A- 0 796 656      US-A- 2 932 673**
**US-A- 5 188 991**

 • DATABASE WPI Section Ch, Week 9444 Derwent
   Publications Ltd., London, GB; Class A41, AN
   94-354712 XP002110931 & JP 06 279396 A
   (NIPPON SHOKUBAI CO LTD), 4 octobre 1994
   (1994-10-04)
 • DATABASE WPI Section Ch, Week 9244 Derwent
   Publications Ltd., London, GB; Class E36, AN
   92-362469 XP002110932 & JP 04 265154 A
   (SUMITOMO METAL MINING CO), 21 septembre
   1992 (1992-09-21)

**Description**

La présente invention concerne le domaine de la thiochimie et plus particulièrement pour objet pour la fabrication de thiols à partir d'alcools.

Les thiols constituent une classe importante de composés organiques, employés comme odorisants, comme additifs pour polymères ou comme intermédiaires pour la synthèse de composés utiles dans divers domaines d'application tels que l'agrochimie, la santé, les cosmétiques, la pétrochimie, les lubrifiants, le traitement des minerais, les matières plastiques, l'odorisation des gaz, l'alimentation animale. Les polythiols sont par ailleurs appelés à connaître un fort développement dans le domaine des additifs pour polymères, des agents de réticulation ou des additifs à haut indice de réfraction pour lentilles organiques et optiques en général.

La synthèse des monothiols se fait généralement, soit par addition d'hydrogène sulfuré sur une liaison carbone-carbone insaturée, soit par substitution du groupement hydroxyle d'un alcool.

L'addition d'hydrogène sulfuré sur un alcène ou un alcyne s'effectue par catalyse hétérogène, le catalyseur solide étant une alumine, une silice-alumine, une zéolithe pure ou dopée, ou une résine sulfonique. La réaction, généralement réalisée à une température comprise entre la température ambiante et 350°C, nécessite l'emploi d'un excès d'agent donneur de soufre pour minimiser les réactions parasites de formation de thioéthers par condensation des thiols formés ou leur addition sur les réactifs. L'activité de ces catalyseurs étant liée à leur caractère acide, ils sont principalement employés pour la fabrication de thiols secondaires ou tertiaires car le groupement SH se retrouve sur le carbone Markovnikov ; cette voie ne permet pas la préparation des thiols primaires avec un rendement suffisant pour une valorisation économique du procédé.

C'est pourquoi la synthèse des thiols primaires en $C_1$ à $C_{12}$ est principalement réalisée par la voie alcool selon la réaction :

$$R\text{-OH} + H_2S \rightarrow R\text{-SH} + H_2O$$

qui est effectuée en phase gaz ou liquide, par catalyse hétérogène. A partir de deux atomes de carbone, les températures d'ébullition des thiols et des alcools sont très voisines de sorte que, pour s'affranchir d'une étape de distillation ultérieure très coûteuse, il est nécessaire d'opérer à conversion totale. La température de réaction étant voisine de 300°C, la déshydratation de l'alcool en oléfine est thermodynamiquement possible et, en présence d'hydrogène sulfuré, l'oléfine résultante est le siège d'une réaction d'addition qui conduit également à la formation d'un thiol selon la réaction suivante :

$$R'\text{-CH} = CH_2 + H_2S \rightarrow R'\text{-CH(SH)-}CH_3$$

En présence d'un catalyseur, cette dernière réaction conduit presque uniquement au thiol secondaire (stabilisation du carbocation secondaire). Il est donc nécessaire d'utiliser des catalyseurs peu déshydratants mais conduisant à des vitesses de thiolation très importantes ; ceux-ci sont principalement constitués par des alumines dopées au sodium ou au potassium ou par des catalyseurs à base d'oxyde de tungstène supporté sur alumine. En plus de cette réaction secondaire, la formation d'éther et de thioéther induit une baisse de rendement en produit désiré.

Les polythiols sont généralement préparés par substitution nucléophile de dérivés halogénés par des sulfhydrates selon la réaction suivante :

$$R(X)_m + mMSH \rightarrow R(SH)_m + mMX$$

où m est un nombre entier égal à 2 ou plus, R est une chaîne hydrocarbonée, aliphatique ou non, X désigne un atome d'halogène (F, Cl ou Br), en général le chlore, et M est un métal alcalin (par exemple Na) ou le cation ammonium.

Cette méthode, utile pour synthétiser de manière sélective des polythiols, est cependant limitée à la synthèse des polythiols primaires ou secondaires, car les réactions de substitution nucléophile sont en général défavorisées lorsque le carbone subissant l'attaque est substitué. Si la mise en oeuvre de cette méthode est facile à l'échelle du laboratoire, elle est difficile au plan industriel en raison, d'une part, de la formation inévitable de sels (MX) non valorisables et, d'autre part, de la sévérité de plus en plus grande des normes environnementales concernant l'emploi des dérivés halogénés. Il apparaît donc souhaitable de développer un procédé catalytique permettant la préparation de thiols (en particulier des polythiols) mettant en oeuvre des alcools (en particulier des polyols) et l'hydrogène sulfuré comme matières premières. L'emploi d'alcools permettra en effet de répondre aux contraintes environnementales imposées par la législation future car, d'une part, ils ne sont pas nocifs et, d'autre part, la réaction forme de l'eau comme sous-produit.

La demande de brevet EP 796 656 concerne un procédé de fabrication de mercaptans à partir d'alcool et de l'hydrogène sulfuré en présence d'un catalyseur comprenant un oxyde inorganique avec un hydroxyde et/ou un oxyde métallique. Le brevet US 2,932,673 décrit des systèmes catalytiques à base d'oxyde de magnésium et d'oxyde de titane ou de zinc pour des réactions de déshydrogénation en phase gaz.

Il a maintenant été trouvé que l'utilisation des catalyseurs à base d'oxyde de zirconium et d'oxyde de magnésium qui sont stables thermiquement en présence d'hydrogène sulfuré et d'eau, pour la thiolation des alcools (en particulier des polyols) est particulièrement avantageuse car ces catalyseurs sont capables de réaliser cette réaction, en phase gaz ou liquide, dans des conditions de température comprises entre 100 et 400°C, et de rapport molaire $H_2S$/fonction alcool compris entre 1 et 10, la pression totale n'excédant pas 20 bars.

Le catalyseur utilisé dans le procédé de l'invention est un oxyde de zirconium dopé par du magnésium ce catalyseur étant préparé, en une seule étape, par coprécipitation d'une solution aqueuse d'un sel de zirconium (chlorure ou nitrate de zirconyle) et d'une solution aqueuse d'un nitrate ou sulfate de magnésium.

Cette coprécipitation, obtenue par ajout d'ammoniaque, conduit à un catalyseur constitué d'oxydes mixtes de zirconium et de magnésium, la teneur massique en magnésium dudit catalyseur pouvant aller de 0,2 à 20 %, de préférence de 1 à 6 %.

La concentration en zirconium dans la solution de sel de zirconium peut aller de 0,01 à 0,4 mole/litre et est de préférence d'environ 0,3 mole/litre.

La concentration en magnésium peut aller jusqu'à une mole/litre et est généralement comprise entre 0,1 et 1, de préférence entre 0,4 et 0,7 mole/litre.

A la solution homogène résultant du mélange de la solution aqueuse de sel de zirconium avec la solution aqueuse de sel de magnésium on ajoute de l'ammoniaque pour obtenir la coprécipitation des oxydes mixtes. On utilise de préférence une solution d'ammoniaque concentrée pour limiter le volume de solution finale, mais la teneur en $NH_4OH$ peut être abaissée sans affecter les propriétés des catalyseurs solides obtenus.

La coprécipitation peut être effectuée à une température allant de 0 à 100°C, mais est de préférence réalisée à la température ambiante (20 à 25°C).

La coprécipitation conduit à un gel qu'il est généralement avantageux de laisser mûrir pendant 5 à 100 heures (de préférence 60 à 80 heures) à une température comprise entre la température ambiante et le point d'ébullition du mélange.

Après décantation, le précipité est lavé à l'eau jusqu'à pH neutre et le solide obtenu est séché à une température comprise entre 50 et 200°C (de préférence environ 120°C) pendant une durée allant de 1 à 24 heures (de préférence environ 12 heures). Ce solide peut être calciné sous gaz inerte (oxygène, azote, ou mélange $N_2$-$O_2$) jusqu'à 800°C sans modification notable de son activité catalytique.

L'alcool de départ mono- ou polyfonctionnel peut être représentée par la formule générale :

$$R(OH)_n$$

dans laquelle n est un nombre entier allant de 1 à 4 (de préférence 1 ou 2), et R est un radical hydrocarbyle de 1 à 30 atomes de carbone choisi parmi les radicaux alkyle, cycloalkyle, aryle, alkylaryle ou aralkyle. Comme exemples non limitatifs de tels alcools on peut mentionner le méthanol, l'éthanol, le n-propanol, l'isopropanol, le n-butanol, l'alcool isobutylique, le tert-butanol, l'alcool amylique, l'hexanol, le cyclo-hexanol, l'octanol, le nonanol, le décanol, le dodécanol, l'alcool benzylique, le phénol, l'éthylène glycol, l'octanediol, le propanetriol et le glycérol.

Le rapport molaire $H_2S$/fonction alcool peut varier dans de larges limites. Il est généralement compris entre 1 et 10, de préférence entre 1 et 3.

La réaction peut être effectuée à une température comprise entre 100 et 400°C.

Le catalyseur peut être utilisé dans un procédé opérant en phase liquide ou en phase gaz, en discontinu ou en continu, selon des techniques bien connues de l'homme de l'art. Le choix du mode opératoire dépend notamment de la nature de l'alcool de départ.

Lorsque l'alcool de départ n'est pas un assez bon solvant de l'hydrogène sulfuré, il peut être avantageux d'opérer au sein d'un solvant inerte tel que, par exemple, un alcane.

## EXEMPLE 1

A 1000 ml d'une solution aqueuse à 0,3 mole/litre de chlorure de zirconyle ($ZrOCl_2.8H_2O$), on a ajouté des quantités variables d'une solution aqueuse à 0,5 mole/litre de nitrate de magnésium [$Mg(NO_3)_2.6H_2O$], cette quantité étant choisie de façon à ce que la teneur pondérale en magnésium des oxydes mixtes de zirconium et de magnésium obtenus varie de 0 à 4,76 %. Les solutions résultantes ayant été placées sous vive agitation, on leur a ajouté à 25°C 90 ml d'ammoniaque à 30 % et les gels obtenus ont été laissés à maturation pendant 72 heures à 25°C.

Après décantation et élimination du liquide surnageant, chaque précipité a été lavé au soxhlet avec de l'eau distillée jusqu'à pH neutre. Les solides ainsi obtenus ont été filtrés et séchés à 120°C pendant 12 heures. Leurs propriétés texturales sont rassemblées dans le tableau 1 où ZrO désigne un catalyseur sans magnésium, non conforme à la présente invention, et les abréviations ZrMg 1, ZrMg 2, ZrMg 3 et ZrMg 4 désignent des catalyseurs selon la présente invention ayant des teneurs variables en magnésium.

La surface spécifique a été déterminée par physisorption d'azote (méthode BET, Norme ASTM D3663).

TABLEAU 1

| Catalyseur | Teneur pondérale (%) en Mg | Caractéristiques | | |
|---|---|---|---|---|
| | | Surface BET $(m^2/g)$ | Rayon moyen poreux (en nm) | Volume poreux (ml/g) |
| ZrO | 0 | 360 | 3 | 0,54 |
| ZrMg 1 | 1,66 | 260 | 2,5 | 0,25 |
| ZrMg 2 | 2,00 | 300 | 2,0 | 0,28 |
| ZrMg 3 | 2,66 | 290 | 2,7 | 0,35 |
| ZrMg 4 | 4,76 | 290 | 2,5 | 0,28 |

Les solides selon l'invention possèdent tous une surface spécifique élevée, même après introduction de magnésium. La distribution poreuse observée est du même ordre de grandeur quel que soit le solide considéré et est tout-à-fait compatible avec une utilisation ultérieure en catalyse.

## EXEMPLE 2

Pour bon nombre de solides de grande surface spécifique, des phénomènes de frittage sont souvent observés lorsque les matériaux sont soumis à un traitement thermique en milieu humide. Comme les catalyseurs selon la présente invention sont utilisés dans des réactions de thiolation, il était primordial de tester leur stabilité thermique en présence d'une atmosphère contenant de l'hydrogène sulfuré et/ou de l'eau.

A cet effet, on a traité des échantillons de solides sous $H_2S$ en présence ou non d'eau par chauffage pendant 4 heures sous flux de gaz à 400°C, cette température étant supérieure à celle généralement utilisée pour la réaction de thiolation.

Les propriétés texturales observées sont rassemblées dans le tableau 2 suivant. On constate que les solides selon l'invention sont stables en présence d'une atmosphère d'$H_2S$ et d'eau.

TABLEAU 2

| Catalyseur | Traitement | Caractéristiques | | |
|---|---|---|---|---|
| | | Surface BET $(m^2/g)$ | Rayon moyen poreux (en nm) | Volume poreux (ml/g) |
| ZrO | Aucun | 360 | 3 | 0,54 |
| | $H_2S$ sec (400°C) | 320 | 3,7 | 0,50 |
| | $H_2S$ + 1% d'eau (400°C) | 302 | 3,5 | 0,48 |
| ZrMg 2 | Aucun | 300 | 2,0 | 0,28 |
| | $H_2S$ sec (400°C) | 285 | 2,2 | 0,30 |
| | $H_2S$ + 1% d'eau (400°C) | 254 | 2,8 | 0,31 |

### EXEMPLE 3 : Thiolation en batch du 1,8-octanediol

L'activité et la sélectivité des catalyseurs selon l'invention vis-à-vis de la synthèse des thiols ont été expérimentées dans la thiolation du 1,8-octanediol.

La réaction a été conduite en réacteur fermé et en milieu triphasique dans les conditions expérimentales suivantes :

- 0,01 mole du 1,8-octanediol
- 75 ml de dodécane
- 0,5 g de catalyseur
- 10 bars de $H_2S$
- 250°C
- agitation : 1500 tr/min.

Le tableau 3 suivant résume les résultats obtenus avec les catalyseurs ZrMg 2 et ZrMg 3 selon l'invention et, à titre comparatif, avec un catalyseur commercial ($K_2WO_4/Al_2O_3$) industriellement utilisé pour ce type de réaction et avec deux catalyseurs ZrO sans magnésium.

On constate que les catalyseurs selon l'invention ont une activité spécifique comparable à celle du catalyseur commercial, mais qu'ils conduisent à une sélectivité en octadithiol multipliée par un facteur de l'ordre de 2,5 ; cette sélectivité étant déterminée à conversion totale, elle correspond directement au rendement en produit désiré.

TABLEAU 3

| Thiolation du 1,8-octanediol en 1,8-octanedithiol | | |
|---|---|---|
| Catalyseur | Activité spécifique ($\mu$mol/g.s.) | Sélectivité en 1,8-octanedithiol à conversion totale |
| $K_2WO_4/Al_2O_3$ | 1,8 | 30 % |
| ZrO non calciné | 1,4 | 58 % |
| ZrO calciné à 400°C | 1,2 | 48 % |
| ZrMg 2 | 1,4 | 73 % |
| ZrMg 3 | 1,3 | 68 % |

### EXEMPLE 4 : Thiolation en continu du 1,6-hexanediol

Le catalyseur ZrMg 2 selon l'invention a été testé dans la thiolation du 1,6-hexanediol, en comparaison avec le catalyseur commercial $K_2WO_4/Al_2O_3$.

La réaction a été conduite en continu dans un réacteur contenant 100 ml de catalyseur en lit fixe. Le 1,6-hexanediol a été introduit dans le réacteur sous forme d'une solution aqueuse à 45% en poids de diol, avec un débit de solution de 66 g/h. Le débit d'$H_2S$ a été réglé de façon à avoir un ratio molaire $H_2S$/diol égal à 10. La température du lit catalytique et la pression ont été maintenues constantes à 330°C et 15 bars absolus respectivement.

Le tableau 4 suivant compare les résultats obtenus avec le catalyseur de référence ($K_2WO_4/Al_2O_3$) et le catalyseur selon l'invention (ZrMg 2).

TABLEAU 4

| Thiolation du 1,6-hexanediol | | | | |
|---|---|---|---|---|
| | | Sélectivités % | | |
| Catalyseur | Conversion % du 1,6-hexanediol | 1-mercapto-6-hexanol + 1,6-hexanedithiol | oléfines | produits lourds |
| $K_2WO_4/Al_2O_3$ | 99,2 | 69,6 | 26,3 | 3,1 |
| ZrMg 2 | 99,6 | 77,2 | 21,1 | 1,7 |

Les résultats obtenus montrent clairement qu'à conversion équivalente et quasi totale, le catalyseur selon l'invention est plus sélectif en produit de thiolation que le catalyseur de référence.

**EXEMPLE 5 : Thiolation en continu du méthanol**

Le catalyseur ZrMg 2 selon l'invention et le catalyseur commercial $K_2WO_4/Al_2O_3$ ont été testés dans la thiolation du méthanol.

La réaction a été conduite en continu dans les conditions suivantes :

- pression atmosphérique
- volume de catalyseur en lit fixe : 200 ml
- débit d'alimentation en méthanol : 237 g/h/litre de catalyseur
- débit d'alimentation en gaz acide ($H_2S/CO_2$ 80/20 en volume) tel que le ratio molaire $H_2S$/méthanol soit égal à 1,5.
- température moyenne du lit catalytique égale à 380°C.

Les résultats, rassemblés dans le tableau suivant, montrent qu'à conversion quasi équivalente, le catalyseur selon l'invention est non seulement plus sélectif en produits de thiolation que le catalyseur de référence, mais également qu'il favorise la formation du méthylmercaptan (MeSH) par rapport au diméthylsulfure qui peut être non désiré.

TABLEAU 5

| Thiolation du méthanol | | | | | |
|---|---|---|---|---|---|
| Catalyseur | Conversion % du méthanol | Productivités en g/heure/litre de catalyseur | | | |
| | | MeSH | DMS (diméthylsulfure) | DME (diméthyléther) | produits de craquage |
| $K_2WO_4/Al_2O_3$ | 92,8 | 211,6 | 48,6 | 19,4 | 1,2 |
| ZrMg 2 | 90,2 | 241,8 | 38,8 | 4,1 | 5,0 |

**Revendications**

1. Procédé de fabrication d'un thiol par mise en contact d'un alcool avec de l'hydrogène sulfuré, **caractérisé en ce que** l'on opère en présence d'un catalyseur à base d'oxydes mixtes de zirconium et de magnésium dont la teneur massique en magnésium peut aller de 0,2 à 20 %, et qui est préparé par coprécipitation d'une solution aqueuse d'un sel de zirconium et d'une solution aqueuse d'un nitrate ou sulfate de magnésium et séchage du gel à une température comprise entre 50 et 200°C.

2. Procédé selon la revendication 1, **caractérisé en ce que** la teneur massique en magnésium est comprise entre 1 et 6 %.

3. Procédé selon la revendication 1 ou 2, **caractérisé en ce que** le sel de zirconium est le chlorure ou le nitrate de zirconyle.

4. Procédé selon l'une des revendications 1 à 3, **caractérisé en ce que** la coprécipitation est effectuée au moyen d'ammoniaque.

5. Procédé selon l'une des revendications 1 à 4, **caractérisé en ce que** la concentration en zirconium dans la solution de sel de zirconium est comprise entre 0,001 et 0,4 mole/litre, de préférence, voisine de 0,3 mole/litre.

6. Procédé selon l'une des revendications 1 à 5, **caractérisé en ce que** la concentration en magnésium est comprise entre 0,1 et 1 mole/litre, de préférence entre 0,4 et 0,7 mole/litre.

7. Procédé selon l'une des revendications 1 à 6, **caractérisé en ce que** la coprécipitation est réalisé à température ambiante.

8. Procédé selon l'une des revendications 1 à 7, **caractérisé en ce que**, avant séchage, on laisse mûrir le gel à une température comprise entre la température ambiante et le point d'ébullition du mélange.

9. Procédé selon l'une des revendications 1 à 8 dans lequel l'alcool de départ répond à la formule générale :

$$R(OH)_n$$

dans laquelle n est un nombre entier allant de 1 à 4 (de préférence 1 ou 2), et R est un radical hydrocarbyle de 1 à 30 atomes de carbone.

10. Procédé selon l'une des revendications 1 à 9 dans lequel le rapport molaire $H_2S$/fonction est compris entre 1 et 10, de préférence entre 1 et 3.

11. Procédé selon l'une des revendications 1 à 10 dans lequel la mise en contact de l'alcool avec l'hydrogène sulfuré est effectuée à une température comprise entre 100 et 400°C.

12. Procédé selon l'une des revendications 1 à 11 dans lequel l'alcool de départ est un polyol.

13. Procédé selon l'un des revendications 1 à 11 dans lequel l'alcool de départ est le méthanol.


**Patentansprüche**

1. Verfahren zur Herstellung eines Thiols, indem ein Alkohol mit schwefelhaltigem Wasserstoff in Berührung gebracht wird, **dadurch gekennzeichnet, dass** die Bearbeitung in Gegenwart eines Katalysators mit gemischten Zirkonium- und Magnesiumoxiden erfolgt, dessen Magnesiummassegehalt von 0,2 bis 20% reichen kann und der durch Mitfällung einer wäßrigen Lösung eines Zirkoniumsalzes und einer wäßrigen Lösung eines Magnesiumnitrats oder Magnesiumsulfats und Trocknung des Gels bei einer Temperatur zwischen 50 und 200 °C zubereitet wird.

2. Verfahren nach Anspruch 1, **dadurch gekennzeichnet, dass** der Magnesiummassegehalt zwischen 1 und 6% beträgt.

3. Verfahren nach Anspruch 1 oder 2, **dadurch gekennzeichnet, dass** das Zirkoniumsalz Zirkoniumchlorid oder Zirkoniumylnitrat ist.

4. Verfahren nach einem der Ansprüche 1 bis 3, **dadurch gekennzeichnet, dass** die Mitfällung mittels Ammoniakwasser erfolgt.

5. Verfahren nach einem der Ansprüche 1 bis 4, **dadurch gekennzeichnet, dass** die Zirkoniumkonzentration in der Zirkoniumsalzlösung zwischen 0,001 und 0,4 Mol/Liter, vorzugsweise annähernd 0,3 Mol/Liter beträgt.

6. Verfahren nach einem der Ansprüche 1 bis 5, **dadurch gekennzeichnet, dass** die Magnesiumkonzentration zwischen 0,1 und 1 Mol/Liter, vorzugsweise zwischen 0,4 und 0,7 Mol/Liter beträgt.

7. Verfahren nach einem der Ansprüche 1 bis 6, **dadurch gekennzeichnet, dass** die Mitfällung bei Umgebungstemperatur erfolgt.

8. Verfahren nach einem der Ansprüche 1 bis 7, **dadurch gekennzeichnet, dass** man das Gel bei einer Temperatur zwischen der Umgebungstemperatur und dem Siedepunkt des Gemischs reifen lässt.

9. Verfahren nach einem der Ansprüche 1 bis 8, wobei der Ausgangsalkohol der allgemeinen Formel

$$R(OH)_n$$

entspricht, wobei n eine ganze Zahl von 1 bis 4 (vorzugsweise 1 oder 2) und R ein Hydrocarbylradikal mit 1 bis 30 Kohlenstoffatomen ist.

10. Verfahren nach einem der Ansprüche 1 bis 9, wobei das Molverhältnis $H_2S$/Funktion zwischen 1 und 10, vorzugsweise zwischen 1 und 3 liegt.

11. Verfahren nach einem der Ansprüche 1 bis 10, wobei der Alkohol mit dem schwefelhaltigen Wasserstoff bei einer Temperatur zwischen 100 und 400 °C in Berührung gebracht wird.

**12.** Verfahren nach einem der Ansprüche 1 bis 11, wobei der Ausgangsalkohol ein Polyol ist.

**13.** Verfahren nach einem der Ansprüche 1 bis 11, wobei der Ausgangsalkohol Methanol ist.

**Claims**

**1.** Process for the manufacture of a thiol by bringing an alcohol into contact with hydrogen sulphide, **characterized in that** the reaction is carried out in the presence of a catalyst based on mixed zirconium and magnesium oxides, the content by mass of magnesium of which can range from 0.2 to 20% and which is prepared by coprecipitating an aqueous solution of a zirconium salt and an aqueous solution of a magnesium nitrate or sulphate and drying the gel at a temperature of between 50 and 200°C.

**2.** Process according to Claim 1, **characterized in that** the content by mass of magnesium is between 1 and 6%.

**3.** Process according to Claim 1 or 2, **characterized in that** the zirconium salt is zirconyl chloride or nitrate.

**4.** Process according to one of Claims 1 to 3, **characterized in that** the coprecipitation is carried out by means of aqueous ammonia.

**5.** Process according to one of Claims 1 to 4, **characterized in that** the concentration of zirconium in the zirconium salt solution is between 0.001 and 0.4 mol/litre and is preferably in the region of 0.3 mol/litre.

**6.** Process according to one of Claims 1 to 5, **characterized in that** the concentration of magnesium is between 0.1 and 1 mol/litre, preferably between 0.4 and 0.7 mol/litre.

**7.** Process according to one of Claims 1 to 6, **characterized in that** the coprecipitation is carried out at room temperature.

**8.** Process according to one of Claims 1 to 7, **characterized in that**, before drying, the gel is allowed to mature at a temperature of between room temperature and the boiling point of the mixture.

**9.** Process according to one of Claims 1 to 8, in which the starting alcohol corresponds to the general formula:

$$R(OH)_n$$

in which n is an integer ranging from 1 to 4 (preferably 1 or 2) and R is a hydrocarbyl radical comprising 1 to 30 carbon atoms.

**10.** Process according to one of Claims 1 to 9, in which the $H_2S$/alcohol functional group molar ratio is between 1 and 10, preferably between 1 and 3.

**11.** Process according to one of Claims 1 to 10, in which the operation in which the alcohol is brought into contact with the hydrogen sulphide is carried out at a temperature of between 100 and 400°C.

**12.** Process according to one of Claims 1 to 11, in which the starting alcohol is a polyol.

**13.** Process according to one of Claims 1 to 11, in which the starting alcohol is methanol.